(19) Europäisches Patentamt
European Patent Office
Office européen des brevets

(11) Publication number: **0 115 424**
**B1**

(12) # EUROPEAN PATENT SPECIFICATION

(45) Date of publication of patent specification: **04.11.87**

(51) Int. Cl.$^4$: **C 07 D 207/337**, A 61 K 31/40

(21) Application number: **84300437.5**

(22) Date of filing: **25.01.84**

(54) **Pyrrole-2-acetylamino acid derivatives.**

(30) Priority: **26.01.83 US 461047**
**26.01.83 US 461055**

(43) Date of publication of application:
**08.08.84 Bulletin 84/32**

(45) Publication of the grant of the patent:
**04.11.87 Bulletin 87/45**

(84) Designated Contracting States:
**AT BE CH DE FR IT LI NL SE**

(56) References cited:
**GB-A-1 195 628**
**GB-A-2 115 417**
**US-A-3 865 840**

(73) Proprietor: **McNeilab, Inc.**
**Springhouse Pennsylvania 19477 (US)**

(72) Inventor: **Carson, John Robert**
**551 Rittenhouse Boulevard**
**Norristown Pennsylvania 19403 (US)**
Inventor: **Migdalof, Bruce Howard**
**156 Richardson Road**
**Robbinsville New Jersey 08691 (US)**

(74) Representative: **Jones, Alan John et al**
**CARPMAELS & RANSFORD**
**43 Bloomsbury Square**
**London, WC1A 2RA (GB)**

EP 0 115 424 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention comprises novel acylamino acids and their pharmaceutically acceptable salts of the following formula (I)

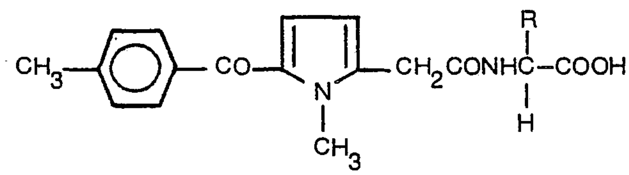

I

wherein R is $C_1$—$C_6$ alkyl, —$(CH_2)_n$ COOH, —$CH_2CH_2SCH_3$, —$(CH_2)_4NH_2$ or —$(CH_2)_2CONH_2$; and n is 1 or 2.

The present invention also provides amide esters of said acids of the following formula (II):

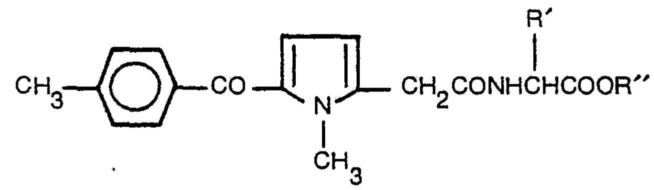

II

wherein: R is H, $C_1$—$C_6$ alkyl, —$(CH_2)_n$ COOR″, —$CH_2CH_2SCH_3$, —$(CH_2)_4$ NHCbz or —$(CH_2)_2$ CONHz; Cbz is benzyloxycarbonyl; n is 1 or 2; and R′ is $C_1$—$C_6$ alkyl or benzyl, provided that when R′ is hydrogen, R″ is not ethyl.

Compounds of the general formula (II) are used as intermediates to make the acids of general formula (I).

Tolmetin (general formula 1 below) is the generic name for the compound: [1-methyl-5-(4-methylbenzoyl)-1*H*-pyrrol-2-yl]-acetic acid. It can also be named as 1-methyl-5-(4-methylbenzoyl)-1*H*-pyrrole-2-acetic acid, has anti-inflammatory activity, and is commercially available as tolmetin sodium dihydrate for use as a nonsteroidal, anti-inflammatory agent. Tolmetin is described and claimed in GB—A—1 195 628 (McNeil Laboratories) and also referred to in US—A—3 865 840 (Carson), which also discloses in Example LXIV the N,N dimethylamide of Tolmetin. Tolmetin is a safe and effective drug, but is administered at relatively large doses. It has been unexpectedly found that the potency of tolmetin can be substantially enhanced and the dosage equipment substantially reduced by means of the novel compounds of the present invention having general formula (I).

GB—A—2 115 417 (Sigma-Tau), which is not part of the prior art in accordance with Article 54 E PC, describes N-mono-substituted and N,N-disubstituted amides of Tolmetin comprising Tolmetin glycinate. This is disclaimed in the present claims.

The particular acid derivatives of tolmetin of this invention are the following: alanine, methionine, glutamic acid, aspartic acid, lysine, and glutamine, either in optically active or racemic form. They may be administered in the form of their free acids or in the form of their pharmaceutically-acceptable salts, for example, as salts of alkali metals, preferably sodium or potassium, or alkaline earth metals, preferably calcium, or salts of organic amines, preferably 2-amino-2-(hydroxymethyl)-1,3-propanediol, (tromethamine). The amino acid derivatives of tolmetin may be prepared by the following reaction scheme (A):

A

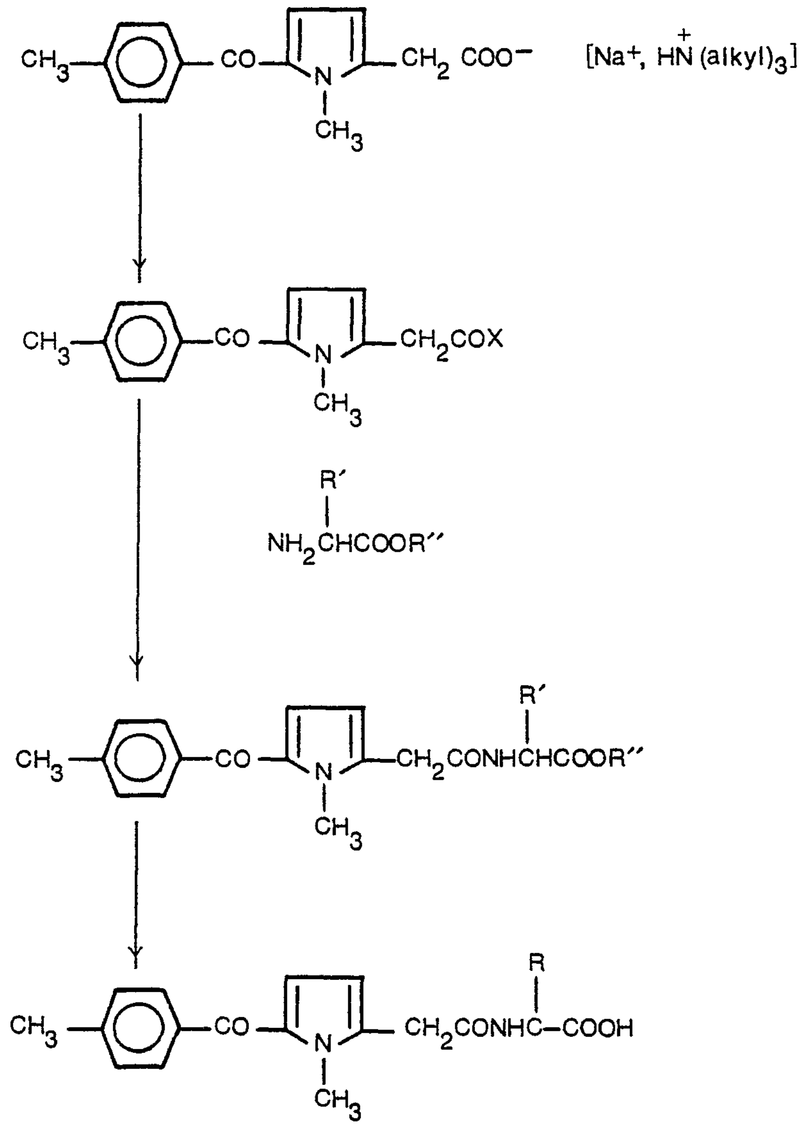

(I) IV V II I

In the above formulae:

X = halide (particularly Cl or Br), $—OCH_2CN$, or —OCOOalkyl

R′ = H,$C_1—_6$ alkyl, $—(CH_2)_nCO_2R''$, $—CH_2CH_2SCH_3$, $—(CH_2)_4NHCbz$, or $—(CH_2)_2CONH_2$

n = 1 or 2

R″ = $C_1—_6$ alkyl or benzyl

R = $C_1—_6$ alkyl, $—(CH_2)_nCO_2H$, $—CH_2CH_2SCH_3$, $—(CH_2)_4NH_2$, or $—(CH_2)_2CONH_2$

$C_1—C_6$ alkyl can be a straight or branched chain, primary, secondary or tertiary, such as methyl, isopropyl, isobutyl, sec-butyl or hexyl.

Compounds of type (I) may be prepared by the illustrated three-step procedures according to scheme A. Tolmetin (1) is first converted to an activated derivative (IV). Three different types of activated derivatives may be used, namely, the acid of halide (X = halide), the activated ester (X = $—OCH_2CN$), or the mixed carbonic anhydride (X = O-CO-O-alkyl). Preparation of acid halides has been previously described by J. R. Carson in US—A— 3,752,826 (1973). The activated ester (X = $OCH_2CN$) may be prepared by reaction of a tolmetin salt with chloroacetonitrile in a dipolar aprotic solvent for example, DMSO, DMF, acetonitrile or acetone) at 20° to 80°C. The mixed anhydrides (X = —O-COO-alkyl) may be prepared by reaction of a tolmetin salt (for example, triethylammonium or N-methylmorpholinium) with an alkyl chloroformate (for example, ethyl chloroformate or isobutyl chloroformate) in an inert aprotic solvent (such as THF or methylene chloride) at −50° to −70°C.

In the second step, amino acid esters ($NH_2—CHR'—CO_2R''$) are acylated by the action of an activated tolmetin derivative (IV) to give amide esters (II). The amino acid esters may be liberated from their corresponding salts by treatment with an organic tertiary amine such as triethylamine or an inorganic weak base such as an alkali metal bicarbonate or carbonate. Acylations of the amino acid esters using tolmetin acyl halides are carried out in the presence of a hydrogen acceptor such as a tertiary amine (e.g., triethylamine) in an inert aprotic solvent at 0° to 80°C. The acylation procedures using the activated esters

(X = —$OCH_2CN$) are carried out in an inert aprotic solvent (for example, THF or dioxane) at elevated temperature (50° to 100°C), preferably in the presence of a weak organic acid catalyst, such as acetic acid. The amino acid esters are used in excess. The acylation reactions utilizing the mixed anhydride reagants (X = —O—COO-alkyl) are carried out in inert aprotic solvents, preferably in the same solution in which the reagent is prepared. The temperature range for carrying out acylations using mixed anhydrides is 0° to −75°C. When the amino acid ester being used is a glutamic acid ester, the mixed anhydride route is preferred, and the activated ester route is not used.

The conversion of amide esters (II) to amide acids (I), may be carried out by conventional saponification using one equivalent of an alkali metal hydroxide in aqueous or mixed aqueous organic solution (e.g. ethanol-water) over a temperature of 25° to 100°C. When R″ is benzyl, the generation of (I) may be carried out by catalytic debenzylation using hydrogen and a noble metal catalyst (for example, palladium or platinum) in an organic solvent such as a lower alkanol or acetic acid. When R″ is benzyl, the generation of (I) may be carried out by treatment with boron tribromide in an inert organic solvent such as methylene chloride.

When R′ is —$(CH_2)_4NHCbZ$ in (II), (CbZ = benzyloxycarbonyl) the removal of the CbZ blocking group is carried out by catalytic debenzylation or preferably by treatment with boron tribromide.

When R″ is tertiary alkyl the conversion of (II) to (I) may be carried out by the action of an aprotic acid, for example by refluxing trifluoroacetic acid.

Alternatively, compounds of type (I) may be prepared by Schotten-Baumann reaction of the acid halide (IV), (X = halide) with an amino acid in the presence of a weak base, preferably sodium or potassium bicarbonate, in aqueous solution as in the following reaction scheme B:

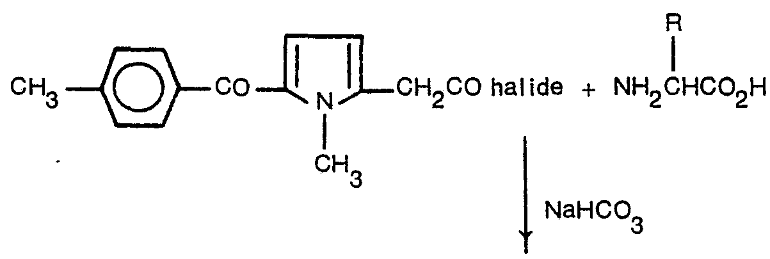


(I)

The salts of the compounds of general formula (I) may be prepared by the conventional method of reacting the acid compounds (I) with the desired base, or by cation exchange.

The anti-inflammatory activity of the compounds 4 of the instant invention was measured in the rat adjuvant arthritis test (S. Wong et al., J. Pharmacol. and Exp. Ther. 185, 127 (1973)). Female Wistar/Lewis rats were given an injection of heat-killed Mycobacterium butyricum in light mineral oil. After eleven days, the animals which had developed an arthritic condition were selected and given daily oral doses of test drug for seventeen days. The compound tested was administered either as aqueous solution of sodium salt (Admin. Form "a") or an aqueous suspension of free acid in 0.05% methyl cellulose (Amin. Form "b"). Paw volumes were measured and the percent inhibition of swelling of the noninjected hind paws compared to controls was calculated for the fourt and seventeenth day after initiation of dosing. The test results are set forth in Table I and are expressed as $ED_{50}$ (mg/kg/day).

TABLE I*

| Compound | Admin. Form | $ED_{50}$ (4 days) | $ED_{50}$ (17 days) |
|---|---|---|---|
| Tolmetin | a | 47.7 | 19.8 |
| (I), R = —$CH_3$ | b | 28.86 | 9.68 |
| (I), R = $(CH_2)_2SCH_3$ | b | 22.07 | 8.66 |
| (I), R = $CH_2CO_2H$ | a | 28.65 | 7.71 |
| (I), R = —$(CH_2)_2CO_2H$ | a | 24.56 | 7.69 |
| (I), R = —$(CH_2)CONH_2$ | a | 31.1 | 11.4 |
| (I), R = —$(CH_2)_4NH_2$ | a | 27.16 | — |

* All test compounds were significantly different from Tolmetin, p ≤ 0.05.

In addition to their anti-inflammatory activity, the compounds of Formula (I) were evaluated for their ability to induce gastric ulceration since gastric irritation is the most significant side effect following administration of nonsteroidal anti-inflammatory agents. The compound tested was administered either as aqueous solution of sodium salt (Admin. Form "a") or an aqueous suspension of free acid in 0.05% methyl

cellulose (Admin. Form "b"). As described by Wong et al., the dose ($UD_{50}$, mg/kg/day) required to produce 50 percent ulcerogenic response following 4 days of oral dosing with the test drug was determined. A therapeutic index in respect to gastric ulceration, the "Anti-inflammatory Index" (AII) was calculated.

$$AII = \frac{UD_{50}}{ED_{50}}$$

The test results are set forth in Table II.

TABLE II*

| Compound | Admin. Form | AII |
|---|---|---|
| Tolmetin | a | 4.7 |
| (I), R = $-CH_3$ | b | 13.09 |
| (I), R = $-(CH_2)_2SCH_3$ | b | 25.31 |
| (I), R = $-CH_2CO_2H$ | a | $<34.89$ |
| (I), R = $-(CH_2)_2CO_2H$ | a | $<32.57$ |
| (I), R = $-(CH_2)_2CONH_2$ | a | 43.06 |

* All test compounds were significantly different from Tolmetin, $p \leq 0.05$.

As can be seen, in addition to greater potency than tolmetins, the compounds of Formula (I) also displays a more favorable therapeutic index than the parent drug. In common with other nonsteroidal anti-inflammatory agents, compounds of Formula (I) also possess analgesic activity. The compounds of formula (I) may be administered to humans in the same general manner as tolmetin, except that, since the compounds are more potent, the amount of active drug per unit dose will be less, on the order of 25 mg to 200 mg.

The following examples are intended to illustrate the present invention.

Preparation of starting material:

Cyanomethyl [1-Methyl-5-(4-methylbenzoyl)-1H-pyrrol-2-yl]-acetate

A 132.9 g (1.76 mole) sample of chloroacetronitrile was added to a suspension of 446.8 g (1.6 mole) of dry sodium [1-methyl-5-(4-methylbenzoyl)-1*H*-pyrrol-2-yl]-acetate in one liter of DMF. The mixture was heated at 55°C for 4 hours. The reaction was cooled and water was added. The precipitate was collected and air dried to give 436 g of cyanomethyl [1-methyl-5-(4-methylbenzoyl)-1*H*-pyrrol-2-yl]-acetate (92% yield), m.p. 132.5°C.

Preparation of intermediates according to the invention

Example 1

Methyl N—{[1-Methyl-5-(4-methylbenzoyl)-1H-pyrrol-2-yl]-acetate}glycinate

A mixture of 5.6 g (0.019 mole) of cyanomethyl [1-methyl-5-(4-methylbenzoyl)-1*H*-pyrrol-2-yl]-acetate, 2.34 g (0.019 mole) of glycine methyl ester hydrochloride, 2.6 ml (0.019 mole) of triethylamine, and 0.2 ml of glacial acetic acid in 15 ml of THF was heated under reflux for 3 hours. A second addition of 1.17 g (0.0095 mole) of glycine methyl ester hydrochloride and 1.3 ml (0.0095 mole) of triethylamine was made. The reaction was heated under reflux for 2 hours. A third addition of 1.17 g of glycine methyl ester hydrochloride and 1.3 ml of triethylamine was made. The reaction was heated under reflux for 1 hour. It was cooled and poured into water. The solid was collected and dried to give 6.0 g of white crystalline methyl *N*—{[1-methyl-5-(4-methylbenzoyl)-1H-pyrrol-2-yl]-acetate}glycinate (97% yield) m.p. 149-150°C.

H nmr ($CDCl_3$) δ7.7 (d, J=9), 2H; 7.2 (d, J=9), 1H; 6.7 (d, J=4), 1H; 6.3 (m), 1H; 6.15 (d, J=4), 1H, 4.0 (m), 2H); 3.9 (s), 3H; 3.71 (s) 3H, 3.68 (s), 2H; 2.4 (s), 3H.

Example 2

Using the procedure of Example 1, employing the following amino acid ester salts in place of glycine methyl ester hydrochloride, and allowing them to react with cyanomethyl [1-methyl-5-(4-methylbenzoyl)-1*H*-pyrrol-2-yl]-acetate in the presence of triethylamine, the respective [1-methyl-5-(4-methylbenzoyl)-1*H*-pyrrol-2-yl]-acetyl derivatives of the amino acid esters were prepared.

| Starting Material/Product | Reaction time (hr.) | % yield | m.p. °C |
|---|---|---|---|
| Alanine methyl ester hydrochloride/Methyl *N*-{[1-methyl-5-(4-methyl benzoyl)-1*H*-pyrrol-2-yl] acetyl}-alaninate | 4 | 75 | 171-2 |
| Methionine methyl ester hydrochloride/Methyl *N*-{[1-methyl-5-(4-methyl-benzoyl)-1*H*-pyrrol-2-yl]acetyl}-methioninate | 5 | 71 | 102-4 |
| $N^6$-Benzyloxycarbonyl-lysine benzyl ester hydrochloride/Benzyl $N^6$-Benzyl-oxycarbonyl-$N^2$-{[1-methyl-5-(4-methyl-benzoyl)-1*H*-pyrrol-2-yl] acetyl}-lysinate | 22 | 62 | 138-9 |
| Aspartic acid dimethyl ester hydrochloride/Dimethyl *N*-{[1-methyl-5-(4-methyl-benzoyl)-1*H*-pyrrol-2-yl] acetyl}-aspartate | 18 | 65 | 115-7 |
| Aspartic acid dibenzyl ester *N*-{(1-methyl-5-(4-methyl-benzoyl)-1*H*-pyrrol-2-yl] acetyl}-aspartate | 9 | 48 | 123-4 |

Example 3

Following the procedure of Example 1, employing the following amino acid esters, causing them to react with cyanomethyl [1-methyl-5-(4-methylbenzoyl)-1*H*-pyrrol-2-yl]-acetate in the presence of triethylamine, the corresponding [1-methyl-5-(4-methylbenzoyl)-1*H*-pyrrol-2-yl]-acetyl amino esters may be prepared respectively:

| Amino Acid Ester | Product |
|---|---|
| L-Valine methyl ester hydrochloride | Methyl *N*-{[1-methyl-5-(4-methyl-benzoyl)]-1*H*-pyrrol-2-yl]acetyl}-valinate |
| L-Leucine methyl ester hydrochloride | Methyl *N*{[1-methyl-5-(4-methyl-benzoyl)]-1*H*-pyrrol-2-yl]acetyl}-leucinate |
| L-Isoleucine methyl ester hydrochloride | Methyl *N*-{[1-methyl-5-(4-methyl-benzoyl)]-1*H*-pyrrol-2-yl]acetyl}-isoleucinate |

Example 4

Dimethyl *N*-[-1-Methyl-5-(4-methylbenzoyl)-1*H*-pyrrol-2-yl]acetylglutamate

To a suspension of 45.10 g (0.154 mole) [1-Methyl-5-(4-methylbenzoyl)-1*H*-pyrrol-2-yl]-acetic acid in 230 ml dry THF was added 16.47 ml (0.15 mole) of *N*-methylmorpholine. The mixture was cooled to −65°C. A 14 ml (0.152 moles) sample of ethyl chloroformate in 50 ml dry THF was added dropwise to a reaction mixture while maintaining the temperature at −65°C. The mixture was stirred for one-half hour after the addition was completed.

Meanwhile, 40.0 g (0.20 mole) L-glutamic acid dimethyl ester hydrochloride was partitioned between 175 ml of cold 20% potassium carbonate and 310 ml of THF. The THF was washed with brine, dried over 4A sieves and anhydrous sodium sulfate. (Elapsed time ~10 minutes).

The L-glutamic acid dimethyl ester solution was added to the reaction mixture as quickly as possible maintaining the temperature at −65°C. It was stirred an additional 4 hours. The reaction mixture was poured into 3N hydrochloric acid, extracted into chloroform, washed with sodium bicarbonate solution, water, brine and dried over anhydrous magnesium sulfate. The solvent was removed in vacuo giving a tan solid which upon recrystallization from 2-propanol gave 40.5 g of dimethyl *N*-{[1-methyl-5-(4-methylbenzoyl)-1H-pyrrol-2-yl]-acetyl}-glutamate (65% yield), m.p. 109—111°C, nmr: 7.70, d, (J=8), 2H; 7,25, d, (J—8), 2H; 6.68, d, (J=4), 1H; 6.52, m, 1H; 6.15, d, (J=4), 1H; 4.55, m, 1H, 3.95, s, 2H; 3.74, S, 3H; 3.66, 2s, (coincidental), 6H; 2.2, m, 4H.

Example 5

Reference Example (not claimed)

*N*-{[1-Methyl-5-(4-methylbenzoyl)-1*H*-pyrrol-2-yl]-acetyl}-glycine

A solution of 64 ml (0.032 mole) of 0.5 N sodium hydroxide was added dropwise over 2 hours to a refluxing solution of 10.49 g (0.0318 mole) of methyl *N*-{[1-methyl-5-(4-methylbenzoyl]-1*H*-pyrrol-2-yl]-acetyl}-glycinate in 100 ml of methanol. The methanol was evaporated *in vacuo*. The solution was added to cold 3N HCl. The solid was collected and air dried. It was recrystallized from 2-propanol to give 7.3 g of white crystalline *N*-{[1-methyl-5-(4-methylbenzoyl)-1*H*-pyrrol-2-yl]-acetyl}-glycine (72% yield), m.p. 197—199°C.

Anal. Calc'd for $C_{17}H_{18}N_2O_4$: C, 64.96; H, 5.77;
Found C, 64.98; H, 5.84.

Preparation of compounds according to the invention

Example 6

Following the procedure of Example 5, employing the *N*-{[1-methyl-5-(4-methylbenzoyl)-1*H*-pyrrol-2-yl]-acetyl} derivatives of the aminoacid methyl esters from Examples III and V in place of methyl *N*-{[1-methyl-5-(4-methylbenzoyl)-1*H*-pyrrol-2-yl]-acetyl}-glycinate and employing one equivalent of sodium hydroxide for each saponifiable ester group, the following *N*-{[1-methyl-5-(4-benzoyl)-1*H*-pyrrol-2-yl]-acetyl} derivatives of amino acids were prepared respectively:

| | Compound 4 | | |
|---|---|---|---|
| | R | % Yield | m.p. |
| *N*-{[1-methyl-5-(4-methyl-benzoyl)-1*H*-pyrrol-2-yl]-acetyl}-alanine | $—CH_3$ | 67 | 181—2° |
| *N*-{[1-methyl-5-(4-methyl-benzoyl)-1*H*-pyrrol-2-yl]-acetyl}-methionine | $—(CH_2)_2SCH_3$ | 92 | 162—4° |
| *N*-{[1-methyl-5-(4-methyl-benzoyl)-1*H*-pyrrol-2-yl]-acetyl}-glutamic acid | $—(CH_2)_2CO_2H$ | 21 | 178—80° |
| *N*-{[1-methyl-5-(4-methyl-benzoyl)-1*H*-pyrrol-2-yl]-acetyl}-aspartic acid | $—CH_2CO_2H$ | 52 | 190°d |

Example 7

Following the procedure of Example 5 and employing the *N*-{[1-methyl-5-(4-methylbenzoyl)-1*H*-pyrrol-2-yl]-acetyl}amino acid methyl esters from Example 4, the following products may be obtained respectively:

*N*-{[1-methyl-5-(4-methylbenzoyl)-1*H*-pyrrol-2-yl]-acetyl}-valine
*N*-{[1-methyl-5-(4-methylbenzoyl)-1*H*-pyrrol-2-yl]-acetyl}-leucine
*N*-{[1-methyl-5-(4-methylbenzoyl)-1*H*-pyrrol-2-yl]-acetyl}-isoleucine

Example 8

*N*-{[1-Methyl-5-(4-methylbenzoyl)-1*H*-pyrrol-2-yl]-acetyl}aspartic acid

A solution of 13.3 g (0.024 mole) of dibenzyl *N*-{[1-methyl-5-(4-methylbenzoyl)-1*H*-pyrrol-2-yl]-acetyl}-aspartate in 150 ml of glacial acetic acid was hydrogenated over 1.3 g of 10% palladium on charcoal for 2¼ hours at room temperature under 50 psi of hydrogen. The solvent was evaporated *in vacuo*. The residue was recrystallized from ethanol-water to give 8.4 g of a gummy red solid. The solid was placed in the thimble of a Soxhlet extractor and continuously extracted with moist ether. The solid was collected from the extract and dried. There was obtained 5.1 g of pink *N*-{[1-methyl-5-(4-methylbenzoyl)-1*H*-pyrrol-2-yl]-acetyl}-aspartic acid (63% yield), m.p. 158—60°.

Anal. Calcd. for $C_{19}H_{20}N_2O_6$: C, 61.28; H, 5.41; N, 7.52
Found: C, 61.18; H, 5.45; N, 7.52

Example 9

$N^2$-{[1-Methyl-5-(4-methylbenzoyl)-1*H*-pyrrol-2-yl]-acetyl}-lysine hydrate (2:1)

A 17.2 g (0.028 mole) sample of $N^6$-benzyloxycarbonyl $N^2$-{[1-methyl-5-(4-methylbenzoyl)-1*H*-pyrrol-2-yl]-acetyl}-lysine benzyl ester was dissolved in 170 ml of methylene chloride and the solution cooled to −70°C in a dry ice bath under a nitrogen atmosphere. Then 227 ml of 1 Molar boron tribromide solution in methylene chloride was added dropwise, keeping the temperature of the reaction mixture below −50°C. The reaction was mechanically stirred for 3 hours at −50°C, then allowed to warm to 22°C over a one-hour period. Water was then added dropwise with vigorous stirring. The methylene chloride was evaporated *in vacuo*, and the aqueous mixture basified to pH 12 with sodium hydroxide solution, then treated with 3N hydrochloric acid to pH 7.5. The solid precipitate was filtered cold, then dried using an evaporating dish.

The solid residue was washed several times with hot acetonitrile, then recrystallized from ethanol-water to give $N^2$-{[1-methyl-5-(4-methylbenzoyl)-1*H*-pyrrol-2-yl]-acetyl}-lysine hydrate (2:1), m.p. 231—233°C *d* as a light tan solid; the yield was 10% of theoretical.

nmr ($CD_3OD/D_2O$): δ 7.9—7.0 (q, 4H, J—14Hz); 6.8—6.6 (d, 1H, J=4Hz); 6.3—6.1 (d, 1H, J—4Hz); 3.85 (s, 3H0; 3.75 (s, 2H); 3.4—3.15 (m, 1H); 3.1—2.6 (m, 2H); 2.4 (s, 3H); 2.1—1.0 (m, 6H).

Example 10

$N^2$-{[1-Methyl-5-(4-methylbenzoyl)-1*H*-pyrrol-2-yl]-acetyl}glutamine

A 20 g sample (0.073 mole) of [1-methyl-5-(4-methylbenzoyl)-1*H*-pyrrol-2-yl]-acetyl chloride was added to a solution of 11.5 g (0.079 mole) of glutamine and 29.3 g (0.32 mole) of sodium bicarbonate in 300 ml of water. The solution was stirred for 3 hours. The solid was filtered and discarded. The filtrate was acidified with 3N HCl. The solid was filtered and washed with water. The solid was dissolved in acetone-water (50:50), treated with charcoal and the solvent evaporated *in vacuo*. The solid was extracted with boiling acetone to give 4.3 g of white crystalline $N^2$-{[1-methyl-5-(4-methylbenzoyl)-1*H*-pyrrol-2-yl]-acetyl} glutamine hydrate (10:1) m.p. 194—6°, yield 15.4%.

| | | | |
|---|---|---|---|
| Anal. calcd. for $C_{20}H_{23}N_3O_5 \cdot 0.1\ H_2O$: | C, 62.04; | H, 6.4; | $H_2O$, 0.46 |
| Found: | C, 62.08; | H, 6.01; | $H_2O$, 0.29 |

Example 11

Methyl *N*-{[1-Methyl-5-(4-methylbenzoyl)-1*H*-pyrrol-2-yl]-acetyl}-glycinate

A solution of 14.0 g (0.139 mole) of triethylamine in 350 ml of ethanol free chloroform was added to a suspension of 8.3 g (0.066 mole) of glycine methyl ester hydrochloride in 500 ml of chloroform. The mixture was cooled to 0° and a solution of 16.83 g (0.061 mole) of [1-methyl-5-(4-methylbenzyl)-1*H*-pyrrol-2-yl]-acetyl chloride in 300 ml of chloroform added. The mixture was stirred at room temperature for 90 minutes. The solution was then washed successively with dilute HCl, sodium bicarbonate solution, water and brine. The solution was dried over anhydrous magnesium sulfate and the solvent evaporated *in vacuo*. The solid was dissolved in chloroform-ethyl acetate and filtered through silica gel. The solvent was evaporated and the residue recrystallized twice from ethyl acetate to give 14.9 g (68% yield) of methyl *N*-{[1-methyl-5-(4-methylbenzoyl)-1*H*-pyrrol-2-yl]-acetyl}-glycinate, m.p. 149—150°C.

Reference Example (not claimed)

Example 12

Salts of *N*-{[1-Methyl-5-(4-methylbenzoyl)-1*H*-pyrrol-2-yl]-acetyl}-glycine

(A) A solution of 361 g (1.1 mole) of methyl *N*-{[1-methyl-5-(4-methylbenzoyl)-1*H*-pyrrol-2-yl]-acetyl}-glycinate in 2 l of ethanol and 220 ml 5N NaOH was heated under reflux for 45 minutes. The mixture was cooled and the solid collected and dried to give 303 g of sodium *N*-{[1-methyl-5-(4-methylbenzoyl)-1*H*-pyrrol-2-yl]-acetyl}-glycinate, m.p. 281—284°C (d).

(B) A solution from 12 g (0.038 mole) of *N*-{[1-methyl-5-(4-methylbenzoyl)-1*H*-pyrrol-2-yl]-acetyl}-glycine and 4.6 g of 2-amino-2-(hydroxymethyl-1,3-propanediol in 300 ml 2-propanol was concentrated *in vacuo* and the solid collected and dried. There was obtained 6.0 g of 2-ammonium-2-(hydroxymethyl)-1,3 propanediol *N*-{[1-methyl-5-(4-methylbenzoyl)-1*H*-pyrrol-2-yl]-acetyl}-glycinate hydrate 1:1.65, m.p. 68—73°C.

(C) To a solution of 6.73 g of 2-ammonium-2-(hydroxymethyl)-1,3-propanediol *N*-{[1-methyl-5-(methylbenzoyl)-1*H*-pyrrol-2-yl]-acetyl}-glycinate in 50 ml of water was added a saturated solution of 22.64 g of $CaCl_2$ in water. The solid was collected, washed with water and air dried to give calcium *N*-{[1-methyl-5-(4-methylbenzoyl)-1*H*-pyrrol-2-yl]-acetyl}-glycinate hydrate [1:0.5:1.6], m.p. 267—270°C (d).

Preparation of compounds according to the invention

Example 13

Following the procedures of the foregoing Example 12 and substituting the appropriate *N*-[1-methyl-5-(4-methylbenzoyl)-1*H*-pyrrol-2-yl]-acetyl aminoacid derivative the following salts may be obtained:

Sodium *N*-{[1-methyl-5-(4-methylbenzoyl)-1*H*-pyrrol-2-yl]-acetyl}-alaninate

Sodium *N*-{[1-methyl-5-(4-methylbenzoyl)-1*H*-pyrrol-2-yl]-acetyl}-lysinate

2-Ammonium-2-(hydroxymethyl)-1,3-propanediol *N*-{[1-methyl-5-(4-methylbenzoyl)-1*H*-pyrrol-2-yl]-acetyl}-methioninate

Calcium *N*-{[1-methyl-5-(4-methylbenzoyl)-1*H*-pyrrol-2-yl]-acetyl}-aspartate [1:1].

The compounds of the present invention may be formulated for use as anti-inflammatory agents in any of the commonly used forms, such as tablets, capsules or syrups, by admixing them with any of the commonly used pharmaceutical carriers.

**Claims**

1. A pyrrole-2-acetylamino acid compound of the following formula (I)

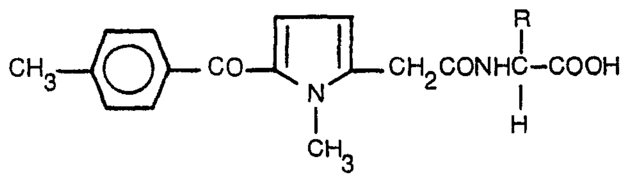

I

or a pharmaceutically acceptable salt thereof, wherein,

R is $C_1$—$C_6$ alkyl, —$(CH_2)_nCOOH$, —$CH_2CH_2SCH_3$, —$(CH_2)_4$ $NH_2$ or —$(CH_2)_2$ $CONH_2$; and

n is 1 or 2.

2. A compound according to claim 1, which is

*N*-{[1-methyl-5-(4-methylbenzoyl)-1*H*-pyrrol-2-yl]-acetyl}-alanine;
*N*-{[1-methyl-5-(4-methylbenzoyl)-1*H*-pyrrol-2-yl]-acetyl}-methionine;
*N*-{[1-methyl-5-(4-methylbenzoyl)-1*H*-pyrrol-2-yl]-acetyl}-aspartic acid;
*N*-{[1-methyl-5-(4-methylbenzoyl)-1*H*-pyrrol-2-yl]-acetyl}-glutamic acid;
*N*-{[1-methyl-5-(4-methylbenzoyl)-1*H*-pyrrol-2-yl]-acetyl}-glutamine; or
*N*[2]-{[1-methyl-5-(4-methylbenzoyl)-1*H*-pyrrol-2-yl]-acetyl}-lysine.

3. An amide ester compound of the following formula (II)

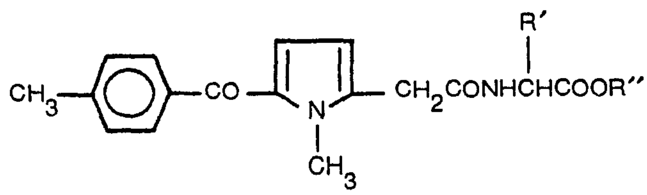

II

wherein:

R′ is H, $C_1$—$C_6$ alkyl, —$(CH_2)_n$ COOR″, —$CH_2CH_2SCH_3$, —$(CH_2)_4NHCbz$, or —$(CH_2)_2CONH_2$;

Cbz is benzyloxycarbonyl;

n is 1 or 2; and

R″ is $C_1$—$C_6$ alkyl or benzyl

provided that when R′ is hydrogen, R″ is not ethyl.

4. A compound according to claim 3, which is:

methyl *N*-{[1-methyl-5-(4-methylbenzoyl)]-1*H*-pyrrol-2-yl]acetyl}-glycinate;
methyl *N*-{[1-methyl-5-(4-methylbenzoyl)-1*H*-pyrrol-2-yl]acetyl}-alaninate;
methyl *N*-{[1-methyl-5-(4-methylbenzoyl)-1*H*-pyrrol-2-yl]acetyl}-methioninate;
benzyl *N*[6]-benzyloxycarbonyl-*N*[2]-{[1-methyl-5-(4-methylbenzoyl)-1*H*-pyrrol-2-yl]acetyl}-lysinate;
dimethyl *N*-{[1-methyl-5-(4-methylbenzoyl)-1*H*-pyrrol-2-yl]acetyl}-aspartate;
dibenzyl *N*-{[1-methyl-5-(4-methylbenzoyl)-1*H*-pyrrol-2-yl]acetyl}-aspartate; or
dimethyl *N*-{[1-methyl-5-(4-methylbenzoyl)-1*H*-pyrrol-2-yl]acetyl}-glutamate.

5. A pharmaceutical composition comprising a compound according to claim 1 or claim 2 in admixture with a pharmaceutically acceptable carrier.

6. A compound according to claim 1 or claim 2 or a composition according to claim 5 for use as an anti-inflammatory agent.

7. A method of producing a compound according to claim 1 or claim 2, comprising hydrolysing an appropriate amide ester according to claim 3 or claim 4.

8. A method of producing an amide ester according to claim 3 or claim 4, which comprises activating a compound of formula III

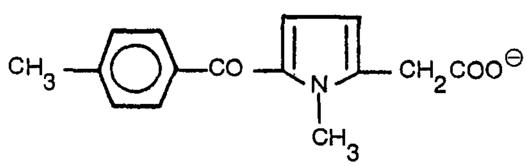

III

to form a compound of formula (IV)

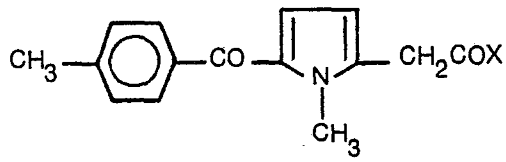

IV

acylating the compound of formula (IV) with a compound of formula (V)

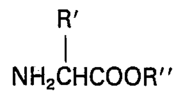

(V)

wherein X is halide, —$OCH_2$—CN, or —OCOO—$C_1$—$C_6$ alkyl, and R′ and R″ are selected appropriately from the definitions given above to produce the desired amide ester.

9. A method according to claim 7, wherein the amide ester is prepared by the method of claim 8.

10. A method of producing a compound according to claim 1 or claim 2, comprising reacting a compound of formula (6).

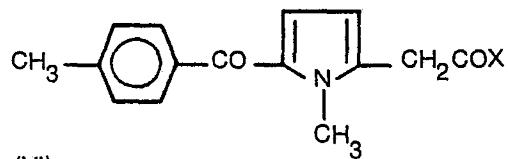

IV

with a compound of formula (VI)

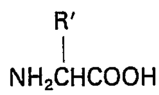

(VI)

by a Schotten-Baumann reaction,
wherein X and R are selected appropriately from the definitions given above to produce the desired compound.

11. A method of producing a composition according to claim 5, comprising mixing a compound according to claim 1 or claim 2 with a pharmaceutically acceptable carrier.

**Patentansprüche**

1. Pyrrol-2-acetylaminosäure-Verbindung der folgenden Formel (I):

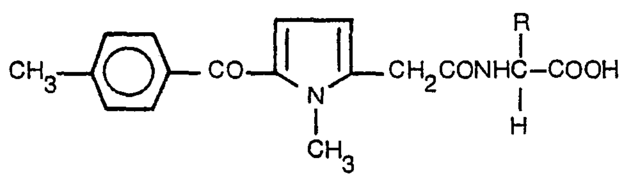

I

oder ein pharmazeutisch annehmbares Salz hievon, worin
R für $C_1$—$C_6$-Alkyl, —$(CH_2)_nCOOH$, —$CH_2CH_2SCH_3$, —$(CH_2)_4NH_2$ oder $(CH_2)_2CONH_2$ steht; und
n den Wert 1 oder 2 hat.

2. Verbindung nach Anspruch 1, nämlich:
*N*-[(1-methyl-5-(4-methylbenzoyl)-1*H*-pyrrol-2-yl)acetyl]-alanin;
*N*-[(1-methyl-5-(4-methylbenzoyl)-1*H*-pyrrol-2-yl)acetyl]-methionin;
*N*-[(1-methyl-5-(4-methylbenzoyl)-1*H*-pyrrol-2-yl)acetyl]-asparaginsäure;
*N*-[(1-methyl-5-(4-methylbenzoyl)-1*H*-pyrrol-2-yl)acetyl]-glutaminsäure;
*N*-[(1-methyl-5-(4-methylbenzoyl)-1*H*-pyrrol-2-yl)acetyl]-glutamin; oder
*N*-[(1-methyl-5-(4-methylbenzoyl)-1*H*-pyrrol-2-yl)acetyl]-lysin.

3. Amid-Ester-Verbindung der folgenden Formel (II):

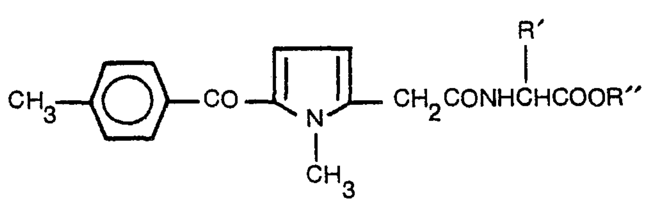

II

worin:

R' für H, $C_1$—$C_6$-Alkyl, —$(CH_2)_nCOOR''$, —$CH_2CH_2SCH_3$, —$(CH_2)_4NHCbz$ oder $(CH_2)_2CONH_2$ steht;
Cbz Benzyloxycarbonyl bedeutet;
n den Wert 1 oder 2 hat; und
R'' für $C_1$—$C_6$-Alkyl oder Benzyl steht,

mit der Maßgabe, daß dann, wenn R' Wasserstoff ist, R'' nicht Ethyl bedeutet.

4. Verbindung nach Anspruch 3, nämlich:
Methyl-*N*-[(1-methyl-5-(4-methylbenzoyl)-1*H*-pyrrol-2-yl)acetyl]-glycinat;
Methyl-*N*-[(1-methyl-5-(4-methylbenzoyl)-1*H*-pyrrol-2-yl)acetyl]-alaninat;
Methyl-*N*-[(1-methyl-5-(4-methylbenzoyl)-1*H*-pyrrol-2-yl)acetyl]-methioninat;
Benzyl-$N^6$-benzyloxycarbonyl-$N^2$-[(1-methyl-5-(4-methylbenzoyl)-1*H*-pyrrol-2-yl)acetyl]-lysinat;
Dimethyl-*N*-[(1-methyl-5-(4-methylbenzoyl)-1*H*-pyrrol-2-yl)acetyl]-aspartat;
Dibenzyl-*N*-[(1-methyl-5-(4-methylbenzoyl)-1*H*-pyrrol-2-yl)acetyl]-aspartat; oder
Dimethyl-*N*-[(1-methyl-5-(4-methylbenzoyl)-1*H*-pyrrol-2-yl)acetyl]-glutamat.

5. Pharmazeutische Zusammensetzung, umfassend eine Verbindung nach Anspruch 1 oder Anspruch 2 im Gemisch mit einem pharmazeutisch annehmbaren Träger.

6. Verbindung nach Anspruch 1 oder 2 oder Zusammensetzung nach Anspruch 5, zur Anwendung als ein entzündungshemmendes Mittel.

7. Verfahren zur Herstellung einer Verbindung nach Anspruch 1 oder Anspruch 2, umfassend ein Hydrolysieren eines entsprechenden Amid-esters nach Anspruch 3 oder 4.

8. Verfahren zur Herstellung eines Amid-esters nach Anspruch 3 oder 4, welches ein Aktivieren einer Verbindung der Formel (III)

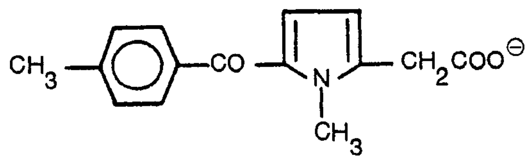
III

zur Ausbildung einer Verbindung (IV)

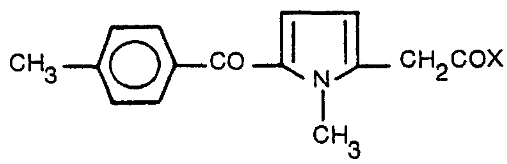
IV

ein Acylieren der Verbindung der Formel (IV) mit einer Verbindung der Formel (V)

R'
|
$NH_2CHCOOR''$ (V)

worin X für Halogenid, —$OCH_2$—CN oder —OCOO—$C_1$—$C_6$-Alkyl steht und R' und R'' in geeigneter Weise aus den vorstehend angegebenen Definitionen ausgewählt sind, zur Ausbildung des gewünschten Amid-esters umfaßt.

9. Verfahren nach Anspruch 7, worin der Amid-ester nach der Methode von Anspruch 8 hergestellt wird.

10. Verfahren zur Herstellung einer Verbindung nach Anspruch 1 oder 2, umfassend ein Umsetzen einer Verbindung der Formel (IV)

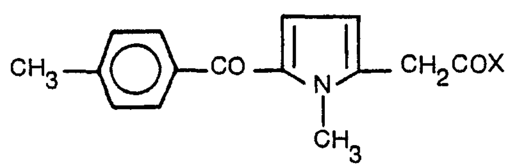
IV

mit einer Verbindung der Formel (VI)

R
|
$NH_2CHCOOH$

nach einer Schotten-Baumann-Reaktion, worin X und R in geeigneter Weise unter den vorstehend angegebenen Definitionen ausgewählt sind, zur Ausbildung der gewünschten Verbindung.

11. Verfahren zur Herstellung einer Zusammensetzung nach Anspruch 5 umfassend ein Vermischen einer Verbindung nach Anspruch 1 oder Anspruch 2 mit einem pharmazeutisch annehmbaren Träger.

**Revendications**

1. Composé de pyrrole-2-acétylaminoacide de formule (I) suivante

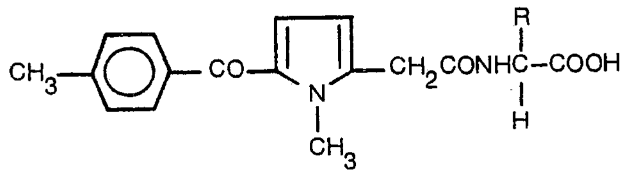

I

ou un de ses sels pharmaceutiquement acceptable, dans lequel R est un groupe alkyle en $C_1$—$C_6$, —$(CH_2)_nCOOH$, —$CH_2CH_2SCH_3$, —$(CH_2)_4NH_2$ ou —$(CH_2)_2CONH_2$, et n est 1 ou 2.

2. Composé selon la revendication 1, qui est
la N-{[1-méthyl-5-(4-méthylbenzoyl)-1H-pyrrole-2-yl)acétyl}-alanine;
la N-{[1-méthyl-5-(4-méthylbenzoyl)-1H-pyrrole-2-yl)acétyl}-méthionine;
l'acide N-{[1-méthyl-5-(4-méthylbenzoyl)-1H-pyrrole-2-yl)acétyl}-aspartique;
l'acide N-{[1-méthyl-5-(4-méthylbenzoyl)-1H-pyrrole-2-yl)acétyl}-glutamique;
la N-{[1-méthyl-5-(4-méthylbenzoyl)-1H-pyrrole-2-yl)acétyl}-glutamine; ou
la N-{[1-méthyl-5-(4-méthylbenzoyl)-1H-pyrrole-2-yl)acétyl}-lysine.

3. Composé d'amide-ester selon la formule (II) suivante

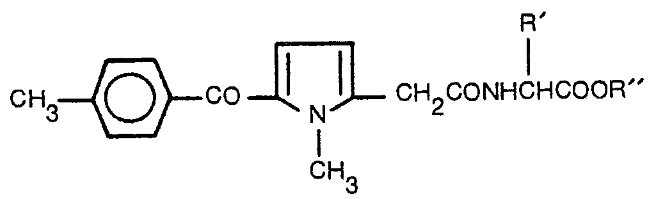

II

dans laquelle R' est H, un groupe alkyle en $C_1$—$C_6$, —$(CH_2)_nCOOR''$, —$CH_2CH_2SCH_3$, —$(CH_2)_4NHCbz$ ou —$(CH_2)_2CONH_2$, Cbz est un groupe benzyloxycarbonyle, n est 1 ou 2, et R'' est un groupe alkyle en $C_1$—$C_6$ ou un groupe benzyle, à condition que, lorsque R' est un hydrogène, R'' ne soit pas un groupe éthyle.

4. Composé selon la revendication 3, qui est
le N-{[1-méthyl-5-(4-méthylbenzoyl)-1H-pyrrole-2-yl]acétyl}-glycinate de méthyle;
le N-{[1-méthyl-5-(4-méthylbenzoyl)-1H-pyrrole-2-yl]acétyl}-alaninate de méthyle;
le N-{[1-méthyl-5-(4-méthylbenzoyl)-1H-pyrrole-2-yl]acétyl}-méthioninate de méthyle;
le $N^6$-benzyloxycarbonyl-$N^2$-{[1-méthyl-5-(4-méthylbenzoyl)-1H-pyrrole-2-yl]acétyl}lysinate de benzyle;
le N-{[1-méthyl-5-(4-méthylbenzoyl)-1H-pyrrole-2-yl]acétyl}-aspartate de diméthyle;
le N-{[1-méthyl-5-(4-méthylbenzoyl)-1H-pyrrole-2-yl]acétyl}-aspartate de dibenzyle; ou
le N-{[1-méthyl-5-(4-méthylbenzoyl)-1H-pyrrole-2-yl]acétyl}-glutamate de diméthyle.

5. Composition pharmaceutique comprenant un composé selon la revendication 1 ou la revendication 2 en mélange avec un véhicule pharmaceutiquement acceptable.

6. Composé selon la revendication 1 ou la revendication 2, ou composition selon la revendication 5, utilisable comme agent anti-inflammatoire.

7. Procédé de production d'un composé selon la revendication 1 ou la revendication 2, comprenant l'hydrolyse d'un amide-ester approprié selon les revendications 3 ou 4.

8. Procédé de production d'un amide-ester selon les revendications 3 ou 4, qui comprend l'activation d'un composé de formule (III)

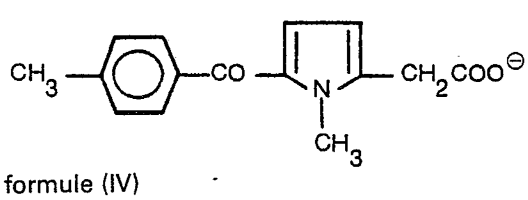

III

pour former un composé de formule (IV)

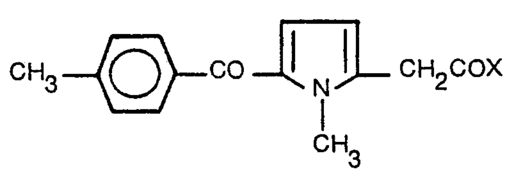

IV

l'acylation du composé de formule (IV) avec un composé de formule (V)

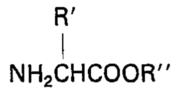

V

dans laquelle X est un halogénure, —$OCH_2CN$ ou —OCOO-(alkyle en $C_1$ à $C_6$), et R' et R'' sont choisis de façon appropriée parmi les définitions indiquées ci-dessus, pour produire l'amide-ester recherché.

9. Procédé selon la revendication 7, dans lequel l'amide-ester est préparé par le procédé selon la revendication 8.

10. Procédé de production d'un composé selon la revendication 1 ou la revendication 2, comprenant la réaction d'un composé de formule (IV)

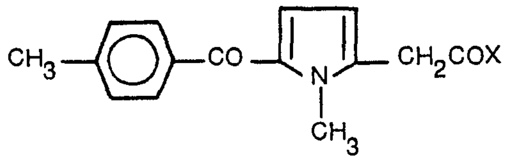

IV

avec un composé de formule (VI)

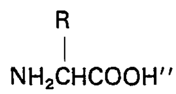

VI

par une réaction de Schotten-Baumann,
dans laquelle X et R sont choisis de façon appropriée parmi les définitions indiquées ci-dessus pour produire le composé recherché.

11. Procédé de production d'une composition selon la revendication 5, comprenant l'opération qui consiste à mélanger un composé selon la revendication 1 ou la revendication 2 avec un véhicule pharmaceutiquement acceptable.